Europäisches Patentamt

European Patent Office

Office européen des brevets

(19)

(11) Veröffentlichungsnummer: **0 041 204**
**B1**

(12) **EUROPÄISCHE PATENTSCHRIFT**

(45) Veröffentlichungstag der Patentschrift:
**14.11.84**

(21) Anmeldenummer: **81103960.1**

(22) Anmeldetag: **22.05.81**

(51) Int. Cl.³: **C 11 C 3/06,** C 07 C 69/30,
C 07 C 67/03

(54) **Verfahren zur kontinuierlichen Herstellung von Fettsäuremonoglyzeriden.**

(30) Priorität: **30.05.80 DE 3020566**

(43) Veröffentlichungstag der Anmeldung:
**09.12.81 Patentblatt 81/49**

(45) Bekanntmachung des Hinweises auf die Patenterteilung:
**14.11.84 Patentblatt 84/46**

(84) Benannte Vertragsstaaten:
**BE CH DE FR GB IT LI NL SE**

(56) Entgegenhaltungen:
**US - A - 2 744 124**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Demmering, Günther, Dr., von Galen-Strasse 40, D-5650 Solingen/Gräfrath (DE)**
Erfinder: **Effey, Gunter, Robert-Koch-Strasse 9, D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die technische Herstellung von Fettsäuremonoglyzeriden – vorzugsweise Glyzerinmonostearat (GMS) – geht von Fetten, bzw. Triglyzeriden aus, die mit Glyzerin zu einem Gemisch von Monoglyzerid und Diglyzerid umgesetzt werden. Technisch wird dabei üblicherweise diskontinuierlich bei Temperaturen von 220 bis 235°C in Gegenwart von Katalysatoren in Rührkesseln gearbeitet, wobei häufig die Verweilzeiten in der Grössenordnung von Stunden liegen. Es ist allgemein üblich, Umesterungsgemische mit einem Gehalt an Monoester zwischen 40 und 45% herzustellen, Monoglyzeridgehalte von 45 bis 50% sind schon als hoch anzusehen.

Das Gleichgewicht der Umesterungsreaktion wird durch höhere Reaktionstemperaturen in Richtung auf die Bildung des Monoglyzerids verschoben, siehe hierzu beispielsweise «Chemie-Technik», 1979, Seiten 343 bis 345. Nachdem diese Abhängigkeit der Monoglyzeridbildung von der Reaktionstemperatur erkannt war, wurde immer wieder versucht, durch Temperaturerhöhung die GMS-Konzentration zu erhöhen. Bedingt durch die bei Temperaturen oberhalb 240°C einsetzende Rückreaktion und aufgrund der Temperaturempfindlichkeit der Reaktionspartner – insbesondere in Anwesenheit der meist alkalisch reagierenden Katalysatoren – führten diese Versuche jedoch stets zu einer Ergebnisverschlechterung. Die Monoglyzeridgehalte des Reaktionsprodukts fielen auf Werte unter 45%, gleichzeitig waren erhebliche Farb- und Geruchsverschlechterungen in Kauf zu nehmen.

Der Erfindung liegt die Aufgabe zugrunde, Verfahrensbedingungen zu entwickeln, unter denen die Umesterung von Triglyzeriden bzw. Fetten mit Glyzerin unter Bildung von Fettsäuremonoglyzeriden in verbesserter Weise und insbesondere mit erhöhten Ausbeuten verläuft. Die Erfindung will weiterhin gleichzeitig die Herstellung hochwertiger, insbesondere hochreiner Fettsäuremonoglyzeride ohne wesentliche Bildung von beispielsweise farblich störenden Reaktionsnebenprodukten ermöglichen. Aufgabe der Erfindung ist weiterhin die Schaffung eines solchen Verfahrens im kontinuierlichen Durchgang des umzuesternden Reaktionsgemisches, wobei mit vergleichsweise kurzen Verweilzeiten gearbeitet werden kann.

Gegenstand der Erfindung ist dementsprechend ein Verfahren zur kontinuierlichen Herstellung von Fettsäuremonoglyzeriden durch Umesterung von Triglyzeriden mit Glyzerin in Gegenwart alkalischer Katalysatoren bei Temperaturen über 200°C. Das erfindungsgemässe Verfahren ist dadurch gekennzeichnet, dass man die Umsetzung in einem statische Mischelemente aufweisenden Rohrreaktor eines Länge/Durchmesserverhältnisses von 100 bis 1000 mit turbulent strömendem Reaktionsgemisch bei einer Reynoldszahl oberhalb 3000 und einer modifizierten Froude-Zahl oberhalb 0,01 mit einem Mischungsverhältnis von Fett/Glycerin im Bereich von 1/2,5 bis 5 Mol/Mol bei Temperaturen von 280 bis 330°C und mittleren Verweilzeiten von 1 bis 3 Minuten durchführt. Das erfindungsgemässe Verfahren ist insbesondere zur Herstellung von Glycerinmonostearat (GMS) durch Umesterung von überwiegend Stearinsäure enthaltenden Fetten mit Glycerin geeignet.

Die erfindungsgemässe Wahl des Rohrreaktors zur Durchführung der an sich bekannten Umesterung führt zu überraschend verbesserten Verfahrensergebnissen in mehrfacher Hinsicht. Besonders auffallend sind die möglichen Ausbeuteverbesserungen an Fettsäuremonoglyzerid. Diese gewünschte Reaktionskomponente kann im Reaktionsgemisch in Ausbeuten deutlich über 50 Gew.-% erhalten werden, in den bevorzugten Ausführungsformen des erfindungsgemässen Verfahrens liegt die Ausbeute an Monoglyzerid oberhalb von 60 Gew.-%, beispielsweise im Bereich von 60 bis 65 Gew.-%. Diese Ausbeuteverbesserung wird durch den Einsatz extrem hoher Reaktionstemperaturen erzielt, die oberhalb 280°C liegen. Gleichwohl sind die erfindungsgemässen Verfahrensprodukte frei von unerwünschten Zersetzungsprodukten des Reaktionsgemisches und farblich einwandfrei. Möglich wird dieses Ergebnis durch die Verknüpfung der hohen Reaktionstemperatur mit kurzen, mittleren Verweilzeiten unter den Bedingungen einer optimal turbulenten Pfropfen- bzw. Kolbenströmung des Reaktionsgemisches im Rohrreaktor.

Die beiden Reaktanten Triglyzerid und Glyzerin bilden bei Reaktionstemperatur 2 flüssige miteinander nicht, bzw. nur beschränkt mischbare Phasen deutlich unterschiedlicher Dichte und neigen dementsprechend zur Entmischung. Erfindungsgemäss wird jedoch dafür Sorge getragen, dass das Reaktionsgemisch in optimaler Vermischung der Reaktanten den Rohrreaktor passiert. Einerseits wird hierzu der Rohrreaktor mit Geschwindigkeiten gefahren, die eine turbulente Strömung des Reaktionsgemisches sicherstellt. Die Reynolds-Zahl des Reaktionsgemisches liegt hierzu oberhalb 3000. Zur Verbesserung des Mischungszustandes sind jedoch zusätzlich im Inneren des Reaktors statische Mischelemente vorgesehen, die die radiale Vermischung der Komponenten des Reaktionsgemisches fördern. Diese statischen Mischelemente können den Rohrreaktor kontinuierlich durchsetzen, vorzugsweise wechseln jedoch in mehrfacher Wiederholung Mischelementsegmente mit solchen Abschnitten des Rohrreaktors ab, in denen keine Mischelemente vorgesehen sind.

Zur Gestaltung des Rohrreaktors und seinem Betrieb in turbulenter Rohrströmung gelten die allgemeinen Angaben des einschlägigen Standes der Technik, vergleiche hierzu beispielsweise «Verfahrenstechnik» 1970, Seiten 535 bis 543. Das Verhältnis der Länge zum Durchmesser des Rohrreaktors wird zwischen 100 und 1000 gewählt, wobei die Absolutlängen des Rohrreaktors im Bereich bis zu 50 m, gewünschtenfalls aber auch im Bereich bis zu 100 m oder darüber liegen können. Um den erfindungsgemäss gewünschten Umsatz zu erreichen, wird ein solcher Rohrreaktor im turbulenten Strömungsbereich (Reynolds-Zahl oberhalb 3000) und bei einer für die Mischung von Flüssigkeiten unterschiedlicher Dichte charakterisierenden modifizierten Froude-Zahl oberhalb 0,01 betrieben. Zur Definition und Errechnung der modifizierten Froude-Zahl; vergleiche die zitierte Literaturstelle «Verfahrenstechnik» 1970, insbesondere Seite 542, rechte Spalte.

Statische Mischelemente zur Verbesserung der radialen Vermischung eines strömenden Flüssigkeitsgemisches – vorzugsweise ohne gleichzeitige Auslösung einer unerwünschten Rückmischung – sind in Literatur und Praxis bekannt, siehe hierzu beispielsweise «Chem.-Ing.-Techn.» 52, (1980), Seiten 285 bis 291.

Die Reaktionstemperaturen innerhalb des Rohrreaktors liegen vorzugsweise im Temperaturbereich von 290 bis 310°C.

Das erfindungsgemässe Verfahren bevorzugt die Mitverwendung alkalischer Umesterungskatalysatoren, selbst wenn in Abwesenheit von Katalysatoren im Rohrreaktor unter den erfindungsgemässen Bedingungen beträchtliche Umsätze erzielt werden. Als Katalysatoren kommen die Verbindungen des Standes der Technik in Betracht. Geeignet sind beispielsweise Alkalihydroxide oder Erdalkalioxide oder auch Alkalialkoholate. Die Konzentration der Katalysatoren liegt vorzugsweise unterhalb 0,5 Gew.-%, insbesondere unterhalb 0,1 Gew.-%, bezogen auf eingesetztes Reaktionsgemisch. Üblicherweise liegt die Katalysatormenge im Bereich von 0,05 bis 0,1%.

In einer bevorzugten Ausführungsform der Erfindung wird der Katalysator dem vorgeheizten Reaktionsgemisch zugesetzt. Zweckmässig ist es hierbei den Katalysator erst unmittelbar vor dem Eintritt des Reaktionsgemisches in den Rohrreaktor oder beim Eintritt, bzw. in der Anfangsphase des Reaktionsgemisches im Rohrreaktor zuzusetzen. Ziel hierbei ist, die Einwirkungszeit des Katalysators unter den Bedingungen der erhöhten Reaktionstemperatur möglichst kurz zu halten. Der Katalysator kann bei Reaktionstemperatur zugesetzt werden oder bei Temperaturen oberhalb 200°C, vorzugsweise oberhalb 240°C.

In einer weiteren bevorzugten Ausführungsform der Erfindung wird das den Rohrreaktor verlassende Reaktionsgemisch schnellst möglich gekühlt. Jede beliebige Form der Kühlung kann gewählt werden. Besonders intensiv wird durch eine flash-Kühlung unter Verdampfung eines Teils des überschüssigen Glyzerins gekühlt. Die Kühlung unmittelbar nach Verlassen des Reaktors soll zweckmässigerweise auf Temperaturen unterhalb 100°C führen.

Es ist erfindungsgemäss weiterhin bevorzugt, den Katalysatorgehalt des Reaktionsgemisches so rasch wie möglich nach Verlassen des Reaktors in an sich bekannter Weise zu neutralisieren. Geeignet ist hierzu die Verwendung von Mineralsäuren, beispielsweise von Phosphorsäure. Im Rahmen des erfindungsgemässen Verfahrens ist es möglich, Kühlung und Neutralisierung miteinander zu verbinden. Durch die spontane Kühlung und Ausschaltung der Katalysatorwirkung wird eine mögliche Rückreaktion des Fettsäuremonoglyzerids in Di- und Triglyzeride und eine Zersetzung der Reaktionsprodukte vermieden.

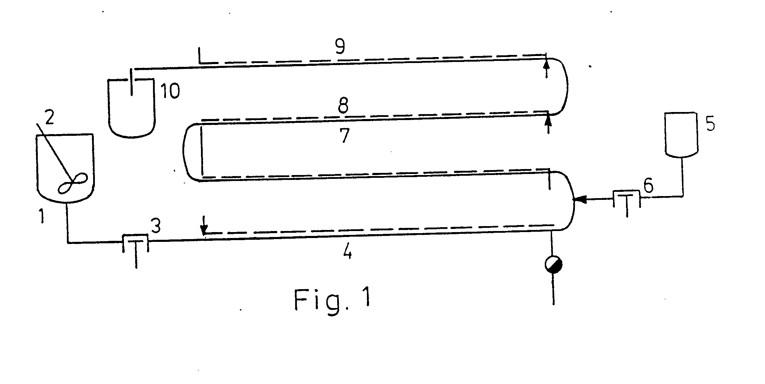

Die nachfolgenden Versuche wurden in einer Versuchsanlage gemäss Fig. 1, die nachfolgend beschrieben ist, durchgeführt:

Die Elemente der Versuchsanlage sind:
Ansatzbehälter (1), Rührer (2), Produkteinsatzpumpe (3), Vorwärmer (4), Katalysatorvorlage (5), Katalysatordosierpumpe (6), Rohrreaktor (7), Wärmeträgeranlage (8), Kühler (9), Produktvorlage (10).

Die Abmessungen des Rohrreaktors betragen:

| Länge | 6 m |
|---|---|
| Innendurchmesser | 22,3 mm |

Es wurden statische Mischelemente mit einem hydraulischen Durchmesser von 2 mm eingesetzt. Die Beheizung des Reaktors erfolgte in einem Doppelmantelrohr durch ein Wärmeträgeröl.

Beispiel 1

10 Teile gehärteter Rindertalg (SZ 4, JZ 1,2) wurden mit 4 Teilen Glyzerin (99,5%ig) bei 90°C vorgemischt.

Bei einem stündlichen Durchsatz von 50 kg Einsatzprodukt und einer Katalysator-Zudosierung – nach dem Wärmeaustauscher (240°C) – von 150 g NaOH (20%ig) wurde die Temperatur des Reaktorinhaltes auf 300°C gebracht.

Nach Abtrennung des nicht gelösten wasserhellen Glyzerins wurde ein Reaktionsprodukt nachfolgender Kennzahlen erhalten:

| Monoester: | 60 Gew.-% |
|---|---|
| Diester: | 24 Gew.-% |
| Triester: | 3 Gew.-% |
| freies Glyzerin: | 13 Gew.-% |
| SZ: | 0,8 |
| Farbzahl 5 ¼″ | 4 g/0,6 r |

Die Aufarbeitung des Reaktionsproduktes kann hier – und ganz allgemein im Rahmen der Erfindung – in an sich bekannter Weise, beispielsweise durch destillative Abtrennung des Glyzerinüberschusses und des gebildeten Monoesters erfolgen, gewünschtenfalls kann aber auch das gebildete Estergemisch als solches zum Einsatz kommen.

Beispiel 2

10 Teile Sojaöl (SZ 0,4) wurden mit 4,5 Teilen Glyzerin (99,5%ig) bei 90° vorgemischt. Bei einem stündlichen Durchsatz von 80 kg Einsatzprodukt und einer Katalysatorzudosierung – bei Reaktionstemperatur von 290°C – von 200 g Natriummethylat (30%) wurde die Reaktion durchgeführt.

Nach Abtrennung des nicht gelösten Glyzerin und Neutralisation mit Phosphorsäure wurde ein rohes Monoglyzerid nachfolgender Kennzahlen erhalten.

| | |
|---|---|
| Monoester: | 57 Gew.-% |
| Diester: | 26 Gew.-% |
| Triester: | 4 Gew.-% |
| Glyzerin: | 13 Gew.-% |
| SZ: | 0,2 |
| Farbzahl 5 ¼" | 3 g/0,5 r |

## Patentansprüche

1. Verfahren zur kontinuierlichen Herstellung von Fettsäuremonoglyzeriden und insbesondere Glyzerinmonostearat durch Umesterung von Triglyzeriden mit Glyzerin in Gegenwart alkalischer Katalysatoren bei Temperaturen über 200°C, dadurch gekennzeichnet, dass man die Umsetzung in einem statische Mischelemente aufweisenden Rohrreaktor eines Länge/Durchmesserverhältnisses von 100 bis 1000 mit turbulent strömendem Reaktionsgemisch bei einer Reynoldszahl oberhalb 3000 und einer modifizierten Froude-Zahl oberhalb 0,01 mit einem Mischungsverhältnis von Fett/Glyzerin im Bereich von 1/2,5 bis 5 Mol/Mol bei Temperaturen von 280 bis 330°C und mittleren Verweilzeiten von 1 bis 3 Minuten durchführt.

2. Verfahren nach Anspruch 1, dadurch gekennzeichnet, dass man bei Reaktionstemperaturen von 290 bis 310°C arbeitet.

3. Verfahren nach Ansprüchen 1 bis 2, dadurch gekennzeichnet, dass man den Katalysator dem vorgeheizten Reaktionsgemisch, vorzugsweise erst unmittelbar vor oder bei dessen Eintritt in den Rohrreaktor zumischt.

4. Verfahren nach Ansprüchen 1 bis 3, dadurch gekennzeichnet, dass das Reaktionsgemisch unmittelbar nach Verlassen des Reaktors auf Temperaturen unterhalb 100°C gekühlt wird.

5. Verfahren nach Ansprüchen 1 bis 4, dadurch gekennzeichnet, dass der Katalysatorgehalt des Reaktionsgemisches unmittelbar nach Verlassen des Reaktors neutralisiert wird, wobei die Kühlung des Reaktionsgemisches und die Katalysatorneutralisation miteinander verbunden werden können.

## Claims

1. A process for the continuous production of fatty acid monoglycerides and, more particularly, glycerine monostearate by transesterifying triglycerides with glycerine in the presence of alkaline catalysts at temperatures above 200°C, characterized in that the reaction is carried out for an average of 1 to 3 minutes at 280 to 330°C in a tube reactor comprising static mixing elements and having a length-to-diameter ratio of from 100 to 1000 with a turbulently flowing reaction mixture in which the fat to glycerine ratio is in the range from 1:2.5 to 5 moles/mole for a Reynold's number above 3000 and a modified Froude number above 0.01.

2. A process as claimed in Claim 1, characterized in that it is carried out at reaction temperatures in the range from 290 to 310°C.

3. A process as claimed in Claims 1 and 2, characterized in that the catalyst is added to the preheated reaction mixture preferably immediately before or during its entry into the tube reactor.

4. A process as claimed in Claims 1 to 3, characterized in that the reaction mixture is cooled to temperatures below 100°C immediately after leaving the reactor.

5. A process as claimed in Claims 1 to 4, characterized in that the catalyst content of the reaction mixture is neutralized immediately after leaving the reactor, cooling of the reaction mixture and neutralization of the catalyst optionally being combined with one another.

## Revendications

1. Procédé de fabrication en continu de monoglycérides d'acides gras et en particulier de monostéarate de glycérine par transestérification de triglycérides avec de la glycérine en présence de catalyseurs alcalins à des températures supérieures à 200°C, caractérisé en ce qu'on exécute la réaction dans un réacteur tubulaire présentant des éléments mélangeurs statiques, d'un rapport longueur/diamètre de 100 à 1000, avec un mélange de réaction à écoulement turbulent à un indice de Reynolds supérieur à 3000 et à un indice de Froude modifié supérieur à 0,01, avec un rapport de mélange graisse/glycérine dans l'intervalle de 1/2,5 à 5 moles/mole à des températures de 280 à 330°C et avec des temps de séjour moyens de 1 à 3 minutes.

2. Procédé selon la revendication 1, caractérisé en ce qu'on opère à des températures de réaction de 290 à 310°C.

3. Procédé selon les revendications 1 à 2, caractérisé en ce qu'on mélange le catalyseur avec le mélange de réaction préchauffé, de préférence seulement directement avant ou à son entrée dans le réacteur tubulaire.

4. Procédé selon les revendications 1 à 3, caractérisé en ce qu'on refroidit le mélange de réaction directement après sa sortie du réacteur à des températures inférieures à 100°C.

5. Procédé selon les revendications 1 à 4, caractérisé en ce qu'on neutralise la teneur en catalyseur du mélange de réaction directement après sa sortie du réacteur, le refroidissement du mélange de réaction et la neutralisation du catalyseur pouvant être associés entre e· .

Fig. 1